# EUROPEAN PATENT APPLICATION

(11) **EP 3 312 193 A1**
(43) Date of publication of application: **25.04.2018**
(21) Application number: 16194560.5
(22) Date of filing: 19.10.2016
(51) Int. Cl.: C07K 14/33, A61K 38/16, A61K 8/64, C12N 9/52

(54) **NOVEL RECOMBINANT BOTULINUM NEUROTOXINS WITH ACCELERATED ONSET OF EFFECT**

(71) Applicant: Merz Pharma GmbH & Co. KGaA, 60318 Frankfurt am Main (DE)
(72) Inventor: Hofmann, Fred, 14480 Potsdam (DE); López de la Paz, Manuela, 65835 Liederbach am Taunus (DE); Scheps, Daniel, 14471 Potsdam (DE); Frevert, Jürgen, 10625 Berlin (DE)
(74) Representative: Graf Keyserlingk, Nikolai

(57) **Abstract**

This invention relates to novel recombinant botulinum neurotoxins serotype A exhibiting a faster onset and/or decreased duration of effect and to methods for the manufacture of such recombinant botulinum neurotoxins. These novel recombinant botulinum neurotoxins comprise a modification of the C-terminus of the light chain of the neurotoxin, and the methods comprise the steps of inserting mutations at the C-terminus of the light chain in the nucleic acid sequence coding for a parental botulinum neurotoxin and expression of the recombinant nucleic acid sequence comprising the mutated sequence in a host cell. The invention further relates to novel recombinant single-chain precursor botulinum neurotoxins used in such methods, nucleic acid sequences encoding such recombinant single-chain precursor botulinum neurotoxins, and pharmaceutical compositions comprising the recombinant botulinum neurotoxin with faster onset and/or decreased duration of effect.

## Description

### FIELD OF THE INVENTION

This invention relates to novel recombinant botulinum neurotoxins serotype A exhibiting an accelerated onset and a decreased duration of effect and to methods for the manufacture of such recombinant botulinum neurotoxins. These novel recombinant botulinum neurotoxins comprise at least one amino acid modification, wherein said at least one amino acid modification is located at the C-terminus of the light chain at position 419 to 438. The invention further relates to pharmaceutical compositions comprising said recombinant neurotoxins as well as pharmaceutical compositions comprising said recombinant neurotoxins together with wild type botulinum neurotoxin. The invention further relates to novel recombinant single-chain precursor botulinum neurotoxins and nucleic acid sequences encoding such recombinant single-chain precursor botulinum neurotoxins.

### BACKGROUND OF THE INVENTION

Clostridium is a genus of anaerobe gram-positive bacteria, belonging to the Firmicutes. Clostridium consists of around 100 species that include common free-living bacteria as well as important pathogens, such as *Clostridium botulinum* and *Clostridium tetani*. Both species produce neurotoxins, botulinum toxin and tetanus toxin, respectively. These neurotoxins are potent inhibitors of calcium-dependent neurotransmitter secretion of neuronal cells and are among the strongest toxins known to man. The lethal dose in humans lies between 0.1 ng and 1 ng per kilogram of body weight.

Oral ingestion of botulinum toxin via contaminated food or generation of botulinum toxin in wounds can cause botulism, which is characterised by paralysis of various muscles. Paralysis of the breathing muscles can cause death of the affected individual.

Although both botulinum neurotoxin (BoNT) and tetanus neurotoxin (TxNT) function via a similar initial physiological mechanism of action, inhibiting neurotransmitter release from the axon of the affected neuron into the synapse, they differ in their clinical response. While the botulinum toxin acts at the neuromuscular junction and other cholinergic synapses in the peripheral nervous system, inhibiting the release of the neurotransmitter acetylcholine and thereby causing flaccid paralysis, the tetanus toxin acts mainly in the central nervous system, preventing the release of the inhibitory neurotransmitters GABA (gamma-aminobutyric acid) and glycine by degrading the protein synaptobrevin. The consequent overactivity in the muscles results in generalized contractions of the agonist and antagonist musculature, termed a tetanic spasm (rigid paralysis).

While the tetanus neurotoxin exists in one immunologically distinct type, the botulinum neurotoxins are known to occur in seven different immunogenic types, termed BoNT/A through BoNT/H. Most *Clostridium botulinum* strains produce one type of neurotoxin, but strains producing multiple toxins have also been described.

Botulinum and tetanus neurotoxins have highly homologous amino acid sequences and show a similar domain structure. Their biologically active form comprises two peptide chains, a light chain of about 50 kDa and a heavy chain of about 100 kDa, linked by a disulfide bond. A linker or loop region, whose length varies among different clostridial toxins, is located between the two cysteine residues forming the disulfide bond. This loop region is proteolytically cleaved by an unknown clostridial endoprotease to obtain the biologically active toxin.

The molecular mechanism of intoxication by TeNT and BoNT appears to be similar as well: entry into the target neuron is mediated by binding of the C-terminal part of the heavy chain to a specific cell surface receptor; the toxin is then taken up by receptor-mediated endocytosis. The low pH in the so formed endosome then triggers a conformational change in the clostridial toxin which allows it to embed itself in the endosomal membrane and to translocate through the endosomal membrane into the cytoplasm, where the disulfide bond joining the heavy and the light chain is reduced. The light chain can then selectively cleave so called SNARE-proteins, which are essential for different steps of neurotransmitter release into the synaptic cleft, e.g. recognition, docking and fusion of neurotransmitter-containing vesicles with the plasma membrane. TeNT, BoNT/B, BoNT/D, BoNT/F, and BoNT/G cause proteolytic cleavage of synaptobrevin or VAMP (vesicle-associated membrane protein), BoNT/A and BoNT/E cleave the plasma membrane-associated protein SNAP-25, and BoNT/C cleaves the integral plasma membrane protein syntaxin and SNAP-25.

Clostridial neurotoxins display variable durations of action that are serotype specific. The clinical therapeutic effect of BoNT/A lasts approximately 3 months for neuromuscular disorders and 6 to 12 months for hyperhidrosis. The effect of BoNT/E, on the other hand, lasts less than 4 weeks. The longer lasting therapeutic effect of BoNT/A makes it preferable for certain clinical use compared to the other serotypes, for example serotypes B, C₁, D, E, F, G. One possible explanation for the divergent durations of action might be the distinct subcellular localizations of BoNT serotypes.

The protease domain of BoNT/A light chain localizes in a punctate manner to the plasma membrane of neuronal cells, co-localizing with its substrate SNAP-25. In contrast, the short-duration BoNT/E serotype is cytoplasmic. Membrane association might protect BoNT/A from cytosolic degradation mechanisms allowing for prolonged persistence of BoNT/A in the neuronal cell.

In *Clostridium botulinum,* the botulinum toxin is formed as a protein complex comprising the neurotoxic component and non-toxic proteins. The accessory proteins embed the neurotoxic component thereby protecting it from degradation by digestive enzymes in the gastrointestinal tract. Thus, botulinum neurotoxins of most serotypes are orally toxic. Complexes with, for example, 450 kDa or with 900 kDa are obtainable from cultures of *Clostridium botulinum.*

In recent years, botulinum neurotoxins have been used as therapeutic agents in the treatment of dystonias and spasms. Preparations comprising botulinum toxin complexes are commercially available, e.g. from Ipsen Ltd (Dysport^{®}) or Allergan Inc. (Botox^{®}). A high purity neurotoxic component, free of any complexing proteins, is for example available from Merz Pharmaceuticals GmbH, Frankfurt (Xeomin^{®}).

Clostridial neurotoxins are usually injected into the affected muscle tissue, bringing the agent close to the neuromuscular end plate, i.e. close to the cellular receptor mediating its uptake into the nerve cell controlling said affected muscle. Various degrees of neurotoxin spread have been observed. The neurotoxin spread is thought to depend on the injected amount and the particular neurotoxin preparation. It can result in adverse side effects such as paralysis in nearby muscle tissue, which can largely be avoided by reducing the injected doses to the therapeutically relevant level. Overdosing can also trigger the immune system to generate neutralizing antibodies that inactivate the neurotoxin preventing it from relieving the involuntary muscle activity. Immunologic tolerance to botulinum toxin has been shown to correlate with cumulative doses.

At present, clostridial neurotoxins are still predominantly produced by fermentation processes using appropriate Clostridium strains. However, industrial production of clostridial neurotoxin from anaerobic Clostridium culture is a cumbersome and time-consuming process. Due to the high toxicity of the final product, the procedure must be performed under strict containment. During the fermentation process, the single-chain precursors are proteolytically cleaved by an unknown clostridial protease to obtain the biologically active di-chain clostridial neurotoxin. The degree of neurotoxin activation by proteolytic cleavage varies between different strains and neurotoxin serotypes, which is a major consideration for the manufacture due to the requirement of neurotoxin preparations with a well-defined biological activity. Furthermore, during fermentation processes using Clostridium strains the clostridial neurotoxins are produced as protein complexes, in which the neurotoxic component is embedded by accessory proteins. These accessory proteins have no beneficial effect on biological activity or duration of effect. They can however trigger an immune reaction in the patient, resulting in immunity against the clostridial neurotoxin. Manufacture of recombinant clostridial neurotoxins, which are not embedded by auxiliary proteins, might therefore be advantageous.

Methods for the recombinant expression of clostridial neurotoxins in *E. coli* are well known in the art (see, for example, WO 00/12728, WO 01/14570, or WO 2006/076902). Furthermore, clostridial neurotoxins have been expressed in eukaryotic expression systems, such as in *Pichia pastoris, Pichia methanolica*, *Saccharomyces cerevisiae,* insect cells and mammalian cells (see WO 2006/017749).

Recombinant botulinum neurotoxins may be expressed as single-chain precursors, which subsequently have to be proteolytically cleaved to obtain the final biologically active botulinum neurotoxin. Thus, botulinum neurotoxins may be expressed in high yield in rapidly-growing bacteria as relatively non-toxic single-chain polypeptides.

Furthermore, it might be advantageous to modify botulinum neurotoxin characteristics regarding biological activity, cell specificity, antigenic potential and duration of effect by genetic engineering to obtain recombinant neurotoxins with new therapeutic properties in specific clinical areas. Genetic modification of botulinum neurotoxins might allow altering the mode of action or expanding the range of therapeutic targets.

Botulinum toxin variants exhibiting a decreased duration of effect in neuromuscular tissue than naturally occurring botulinum toxins would be very advantageous for certain indications where a shortened duration of effect is desired.

US 2002/0127247 describes clostridial neurotoxins comprising modifications in secondary modification sites and exhibiting altered biological persistence. WO2011/000929 and WO 2013/068476 disclose modified clostridial neurotoxin with degradation signals in their light chain which reduces the duration of action. WO2013/017522 discloses a modified clostridial neurotoxin with altered duration of action due to an artificially introduced Calpain cleavage site.

There is a strong demand to produce new botulinum neurotoxins serotype A with a decreased duration of effect and with improved properties, in order to allow for exploitation of the therapeutic potential of BoNT serotype A, which have so far been considered impractical for certain clinical application. Ideally, the decreased duration of effect of a particular botulinum neurotoxin serotype A could be adjusted in a tailor-made fashion in order to address any particular features and demands of a given indication, such as the amount of neurotoxin being administered, frequency of administration etc. In addition, it would be desirable to produce botulinum neurotoxins serotype A which exhibit a decreased duration of effect in neuromuscular tissue than naturally occurring botulinum toxins serotype A and / or a faster onset than naturally occurring botulinum toxins serotype A. To date, such aspects have not been solved satisfactorily.

### OBJECTS OF THE INVENTION

It was an object of the invention to provide recombinant botulinum neurotoxins serotype A exhibiting a faster onset and / or a decreased duration of effect in neuromuscular tissue than naturally occurring botulinum toxins. It was also an object of the invention to provide a pharmaceutical or cosmetic composition comprising recombinant botulinum neurotoxins serotype A exhibiting an accelerated onset of effect without a decreased duration of effect in neuromuscular tissue than naturally occurring botulinum toxins. It was a further object of the invention to establish a reliable and accurate method for manufacturing and obtaining such recombinant botulinum neurotoxins. Such a method and novel precursor botulinum neurotoxins used in such methods would serve to satisfy the great need for recombinant botulinum neurotoxins exhibiting fast onset and a decreased duration of effect.

### SUMMARY OF THE INVENTION

The naturally occurring botulinum toxin serotypes display highly divergent durations and onsets of effect, probably due to their distinct subcellular localization. In comparison with BoNT/E the onset of effect of BoNT/A is late. BoNT/A exhibits the longest persistence and was shown to localize in the vicinity of the plasma membrane of neuronal cells, whereas the fast-onset and short-duration BoNT/E serotype is cytosolic. However, additional factors such as degradation, spread or diffusion, and/or translocation rates might have a decisive impact on the differences in the duration and onset of effect for the individual botulinum toxin serotypes.

So far, no generally applicable method for modifying botulinum neurotoxins in order to accelerate the onset and to modify duration of effect is available. Surprisingly, it has been found that recombinant botulinum neurotoxins serotype A having such an effect can be obtained by modifying amino acid residues at position 419 to 438 at the C-terminus of the light chain of botulinum neurotoxin serotype A, and by subsequent heterologous expression of the generated construct in recombinant host cells.

Thus, in one aspect, the present invention relates to recombinant botulinum neurotoxin serotype A comprising at least one amino acid modification, wherein said at least one amino acid modification is located at the C-terminus of the light chain at position 419 to 438 (SEQ ID NO 1), wherein said at least one amino acid modification confers a faster onset and/or a shorter duration of effect than an otherwise identical botulinum neurotoxin lacking said at least one amino acid modification.

In another aspect, the present invention relates to a composition, in particular to a pharmaceutical composition comprising the recombinant botulinum neurotoxin of the present invention. In a further aspect, the present invention relates to a pharmaceutical composition comprising the recombinant botulinum neurotoxin of the present invention together the wild type botulinum neurotoxin A.

In yet another aspect, the present invention relates to the use of the composition of the present invention for cosmetic treatment.

In another aspect, the present invention relates to a method for the generation of the recombinant botulinum neurotoxin of the present invention, comprising the step of obtaining a recombinant nucleic acid sequence encoding a recombinant single-chain precursor botulinum neurotoxin by modifying the nucleic acid sequence at the C-terminus of the light chain of the botulinum neurotoxin serotype A.

In another aspect, the present invention relates to a recombinant single-chain precursor botulinum neurotoxin comprising a modified light chain.

In another aspect, the present invention relates to a nucleic acid sequence encoding the recombinant single-chain precursor botulinum neurotoxin of the present invention.

In another aspect, the present invention relates to a method for obtaining the nucleic acid sequence of the present invention, comprising the step of modifying a nucleic acid sequence encoding the C-terminus of the light chain of the parental botulinum neurotoxin.

In another aspect, the present invention relates to a vector comprising the nucleic acid sequence of the present invention, or the nucleic acid sequence obtainable by the method of the present invention.

In another aspect, the present invention relates to a recombinant host cell comprising the nucleic acid sequence of the present invention, the nucleic acid sequence obtainable by the method of the present invention, or the vector of the present invention.

In another aspect, the present invention relates to a method for producing the recombinant single-chain precursor botulinum neurotoxin of the present invention, comprising the step of expressing the nucleic acid sequence of the present invention, or the nucleic acid sequence obtainable by the method of the present invention, or the vector of the present invention in a recombinant host cell, or cultivating the recombinant host cell of the present invention under conditions that result in the expression of said nucleic acid sequence.

### FIGURES

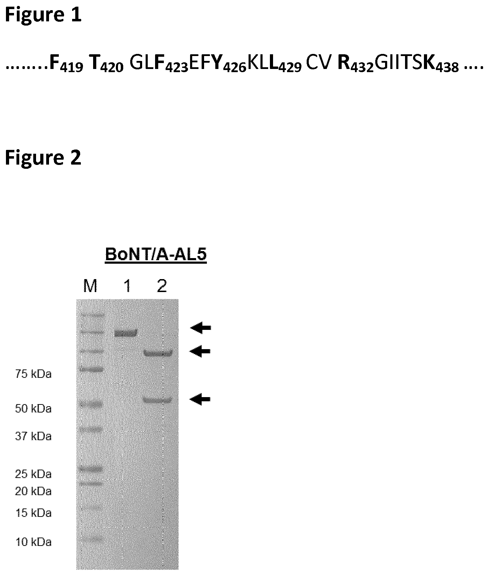

**Figure 1****:** Schematic Presentation of the C-terminus of Botulinum Toxin A, positions to be modified in bold letters
**Figure 2****:** SDS·PAGE of purified BoNT/A-AL5. MW: Molecular weight marker. Lane 2: Prior to applying the samples to the gel, ß-mercaptoethanol was added.
**Figure 3****:** Mouse running assay with BoNT/A-AL5
**Figure 4****:** SDS·PAGE of purified BoNT/A-AL6. MW: Molecular weight marker. Lane 2: Prior to applying the samples to the gel, ß-mercaptoethanol was added.
**Figure 5****:** Mouse running assay with BoNT/A-AL6

### DETAILED DESCRIPTION OF THE INVENTION

The present invention may be understood more readily by reference to the following detailed description of the invention and the examples included therein.

In one aspect, the present invention relates to a recombinant botulinum neurotoxin serotype A or a functional variant thereof comprising at least one amino acid modification, wherein said at least one amino acid modification is located at the C-terminus of the light chain at position 419 to 438 with the amino acid sequence numbering as in GenBank accession number YP_001253342.1 or in SEQ ID NO: 1, wherein said at least one amino acid modification confers a faster onset and/or a shorter duration of effect than an otherwise identical botulinum neurotoxin lacking said at least one amino acid modification.

In the context of the present invention, the term "botulinum neurotoxin" refers to a natural neurotoxin obtainable from bacteria *Clostridium botulinum,* or to a neurotoxin obtainable from alternative sources, including from recombinant technologies or from genetic or chemical modification. Particularly, the botulinum neurotoxins have endopeptidase activity.

Botulinum neurotoxins are produced as single-chain precursors that are proteolytically cleaved by an unknown clostridial endoprotease within the loop region to obtain the biologically active disulfide-linked di-chain form of the neurotoxin, which comprises two chain elements, a functionally active light chain and a functionally active heavy chain, where one end of the light chain is linked to one end of the heavy chain not via a peptide bond, but via a disulfide bond.

In the context of the present invention, the term "botulinum neurotoxin light chain" refers to that part of a botulinum neurotoxin that comprises an endopeptidase activity responsible for cleaving one or more proteins that is/are part of the so-called SNARE-complex involved in the process resulting in the release of neurotransmitter into the synaptic cleft: In naturally occurring botulinum neurotoxins, the light chain has a molecular weight of approx. 50 kDa.

In the context of the present invention, the term "C-terminus of the light" chain comprises to the amino acids AS408-AS438 of BoNT/A.

In the context of the present invention, the term "botulinum neurotoxin heavy chain" refers to that part of a botulinum neurotoxin that is responsible for entry of the neurotoxin into the neuronal cell: In naturally occurring botulinum neurotoxins, the heavy chain has a molecular weight of approx. 100 kDa.

In the context of the present invention, the term "functionally active botulinum neurotoxin chain" refers to a recombinant clostridial neurotoxin chain able to perform the biological functions of a naturally occurring *Clostridium botulinum* neurotoxin chain to at least about 50%, particularly to at least about 60%, to at least about 70%, to at least about 80%, and most particularly to at least about 90%, where the biological functions of botulinum neurotoxin chains include, but are not limited to, binding of the heavy chain to the neuronal cell, entry of the neurotoxin into a neuronal cell, release of the light chain from the di-chain neurotoxin, and endopeptidase activity of the light chain. Methods for determining a neurotoxic activity can be found, for example, in WO 95/32738, which describes the reconstitution of separately obtained light and heavy chains of tetanus toxin and botulinum toxin. Also cell-based assay methods as described for example in WO2009/114748, WO 2013/049508 and WO2014/207109.

In the context of the present invention, the term "about" or "approximately" means within 20%, alternatively within 10%, including within 5% of a given value or range. Alternatively, especially in biological systems, the term "about" means within about a log (i.e. an order of magnitude), including within a factor of two of a given value.

In the context of the present invention, the term "recombinant botulinum neurotoxin" refers to a composition comprising a botulinum neurotoxin that is obtained by expression of the neurotoxin in a heterologous cell such as *E. coli,* and including, but not limited to, the raw material obtained from a fermentation process (supernatant, composition after cell lysis), a fraction comprising a botulinum neurotoxin obtained from separating the ingredients of such a raw material in a purification process, an isolated and essentially pure protein, and a formulation for pharmaceutical and/or aesthetic use comprising a botulinum neurotoxin and additionally pharmaceutically acceptable solvents and/or excipients.

In the context of the present invention, the term "comprises" or "comprising" means "including, but not limited to". The term is intended to be open-ended, to specify the presence of any stated features, elements, integers, steps or components, but not to preclude the presence or addition of one or more other features, elements, integers, steps, components, or groups thereof. The term "comprising" thus includes the more restrictive terms "consisting of" and "consisting essentially of".

In a further aspect, the present invention relates to a recombinant botulinum neurotoxin serotype A or a functional variant thereof comprising at least one amino acid modification, wherein said at least one amino acid modification is located at the C-terminus of the light chain at position 419 to 438 with the proviso that the two leucines at position 427 and 428 remain unchanged, wherein said at least one amino acid modification confers a faster onset and/or a shorter duration of effect than an otherwise identical botulinum neurotoxin lacking said at least one amino acid modification.

In a further aspect, the structural modification in the BoNT/A light chain comprises at least one amino acid modification which is located at the C-terminus of the light chain at at least one position selected from F419, T420, F423, Y426, R432 and K438.

In a further aspect, the structural modification in the BoNT/A light chain comprises one or more modifications selected from the group consisting of a
(i) T420R modification (threonine at position 420 of the light chain is replaced by an arginine),
(ii) T420E modification (threonine at position 420 is replaced by a glutamic acid),
(iii) T420H modification (threonine at position 420 is replaced by histidine),
(iv) F423M modification (phenylalanine at position 423 of the light chain is replaced by an methionine),
(v) F423Y modification (phenylalanine at position 423 of the light chain is replaced by an tyrosine),
(vi) Y426F modification (tyrosine at position 426 of the light chain is replaced by a phenylanine),
(vii) Y426R modification (tyrosine at position 426 of the light chain is replaced by a arginine),
(viii) T420G modification (threonine at position 420 of the light chain is replaced by glycine),
(ix) R432G modification (arginine at position 432 of the light chain is replaced by glycine),
(x) K438G modification (lysine at position 438 of the light chain is replaced by glycine).

In particular embodiments, the recombinant botulinum neurotoxin serotype A comprises three amino acid modifications, wherein these modifications are located at the C-terminus of the light chain at position T420, F423 and Y426, in particular wherein these amino acids are substituted as follows T420R, F423M, Y426F as found in SEQ NO 2.

In particular embodiments, the recombinant botulinum neurotoxin serotype A comprises three amino acid modifications, wherein these modifications are located at the C-terminus of the light chain at position T420, F423 and Y426, wherein these amino acids are substituted as follows T420E, F423M, Y426F as found in SEQ NO 3.

In particular embodiments, the recombinant botulinum neurotoxin serotype A comprises four amino acid modifications, wherein these modifications are located at the C-terminus of the light chain at position T420, Y426, R432, and K438, in particular wherein these amino acids are substituted as follows T420S, Y426F, R432G, K438R as found in SEQ NO 4.

It is encompassed by the scope of the present invention that other and/or further modifications can be introduced into the light chain at position 419 to 438 of a recombinant botulinum neurotoxin serotype A or a functional variant thereof, either in addition to one or more of the modifications mentioned above or instead of these

In the context of the present invention, the term "functional variant of a botulinum neurotoxin" refers to a neurotoxin that differs in the amino acid sequence and/or the nucleic acid sequence encoding the amino acid sequence from a botulinum neurotoxin, but is still functionally active. In the context of the present invention, the term "functionally active" refers to the property of a recombinant botulinum neurotoxin to exhibit a biological activity of at least about 20 %, particularly to at least about 40%, at least about 70%, at least about 80%, and most particularly at least about 90% of the biological activity of a naturally occurring parental botulinum neurotoxin, i.e. a parental botulinum neurotoxin without modifications at the C-terminus of the light chain, where the biological functions include, but are not limited to, binding to the neurotoxin receptor, entry of the neurotoxin into a neuronal cell, release of the light chain from the two-chain neurotoxin, and endopeptidase activity of the light chain, and thus inhibition of neurotransmitter release from the affected nerve cell. In vivo assays for assessing biological activity include the mouse LD50 assay and the ex vivo mouse hemidiaphragm assay as described by Pearce et al. (Pearce 1994, Toxicol. Appl. Pharmacol. 128: 69-77) and Dressler et al. (Dressler 2005, Mov. Disord. 20:1617-1619, Keller 2006, Neuroscience 139: 629-637) or a cell-based assay as described in WO2009/114748, WO2014/207109 or WO 2013/049508. The biological activity is commonly expressed in Mouse Units (MU). As used herein, 1 MU is the amount of neurotoxic component, which kills 50% of a specified mouse population after intraperitoneal injection, i.e. the mouse i.p. LD50.

On the protein level, a functional variant will maintain key features of the corresponding botulinum neurotoxin serotype A, such as key residues for the endopeptidase activity in the light chain, or key residues for the attachment to the neurotoxin receptors or for translocation through the endosomal membrane in the heavy chain, but may contain modifications comprising a substitution of one or more amino acids of the corresponding botulinum neurotoxin.

In another embodiment, the functional variant of a botulinum neurotoxin additionally comprises a signal peptide. Usually, said signal peptide will be located at the N-terminus of the neurotoxin. Many such signal peptides are known in the art and are comprised by the present invention. In particular, the signal peptide results in transport of the neurotoxin across a biological membrane, such as the membrane of the endoplasmic reticulum, the Golgi membrane or the plasma membrane of a eukaryotic or prokaryotic cell. It has been found that signal peptides, when attached to the neurotoxin, will mediate secretion of the neurotoxin into the supernatant of the cells. In certain embodiments, the signal peptide will be cleaved off in the course of, or subsequent to, secretion, so that the secreted protein lacks the N-terminal signal peptide, is composed of separate light and heavy chains, which are covalently linked by disulfide bridges, and is proteolytically active.

In particular embodiments, the functional variant has a sequence identity of at least about 40%, at least about 50%, at least about 60%, at least about 70% or most particularly at least about 80%, and a sequence homology of at least about 60%, at least about 70%, at least about 80%, at least about 90%, or most particularly at least about 95% to the corresponding part in the parental botulinum neurotoxin serotype A. Methods and algorithms for determining sequence identity and/or homology, including the comparison of variants having deletions, additions, and/or substitutions relative to a parental sequence, are well known to the practitioner of ordinary skill in the art. The term "identity" as used herein refers to sequence identity characterized by determining the number of identical amino acids between two nucleic acid sequences or two amino acid sequences wherein the sequences are aligned so that the highest order match is obtained. It can be calculated using published techniques or methods codified in computer programs such as, for example, BLASTP, BLASTN or FASTA (Altschul 1990, J Mol Biol 215, 403). The percent identity values are, in one aspect, calculated over the entire amino acid sequence. A series of programs based on a variety of algorithms is available to the skilled worker for comparing different sequences. In this context, the algorithms of Needleman and Wunsch or Smith and Waterman give particularly reliable results. To carry out the sequence alignments, the program PileUp (Higgins 1989, CABIOS 5, 151) or the programs Gap and BestFit (Needleman 1970, J Mol Biol 48; 443; Smith 1981, Adv Appl Math 2, 482), which are part of the GCG software packet (Genetics Computer Group 1991, 575 Science Drive, Madison, Wisconsin, USA 53711), may be used. The sequence identity values recited above in percent (%) are to be determined, in another aspect of the invention, using the program GAP over the entire sequence region with the following settings: Gap Weight: 50, Length Weight: 3, Average Match: 10.000 and Average Mismatch: 0.000, which, unless otherwise specified, shall always be used as standard settings for sequence alignments. On the DNA level, the nucleic acid sequences encoding the functional homologue and the parental botulinum neurotoxin may differ to a larger extent due to the degeneracy of the genetic code. It is known that the usage of codons is different between prokaryotic and eukaryotic organisms. Thus, when expressing a prokaryotic protein such as a botulinum neurotoxin, in a eukaryotic expression system, it may be necessary, or at least helpful, to adapt the nucleic acid sequence to the codon usage of the expression host cell, meaning that sequence identity or homology may be rather low on the nucleic acid level.

In the context of the present invention, the term "variant" refers to a botulinum neurotoxin that is a chemically, enzymatically, or genetically modified derivative of a corresponding neurotoxin of *C. botulinum* neurotoxin serotype A. A chemically modified derivative may be one that is modified by pyruvation, phosphorylation, sulfatation, lipidation, pegylation, glycosylation and/or the chemical addition of an amino acid or a polypeptide comprising between 2 and about 100 amino acids, including modification occurring in the eukaryotic host cell used for expressing the derivative. An enzymatically modified derivative is one that is modified by the activity of enzymes, such as endo- or exoproteolytic enzymes, including modification by enzymes of the eukaryotic host cell used for expressing the derivative. As pointed out above, a genetically modified derivative is one that has been modified by deletion or substitution of one or more amino acids contained in, or by addition of one or more amino acids (including polypeptides comprising between 2 and about 100 amino acids) to, the amino acid sequence of said botulinum neurotoxin. Methods for designing and constructing such chemically or genetically modified derivatives and for testing of such variants for functionality are well known to anyone of ordinary skill in the art.

In particular embodiments, said recombinant botulinum neurotoxin serotype A comprising at least one amino acid modification, wherein said at least one amino acid modification is located at the C-terminus of the light chain at position 419 to 438 shows a faster onset and / or a decreased duration of effect relative to an identical botulinum neurotoxin without the modifications at the C-terminus of the light chain.

In the context of the present invention, the terms "faster onset of effect" "decreased duration of effect" or "decreased duration of action" refers to a faster induced and a shorter lasting denervation mediated by a botulinum neurotoxin of the present invention. For example, as disclosed herein, administration of a disulfide-linked di-chain botulinum neurotoxin serotype A comprising said modifications at the C-terminus of the light chain results in localized paralysis at an earlier time point and for a shorter period of time relative to administration of an identical disulfide-linked di-chain botulinum neurotoxin serotype A without the modifications at the C-terminus of the light chain.

In the context of the present invention, the term "decreased duration of effect/action" is defined as a more than about 20%, particularly more than about 50%, more particularly more than about 60% decreased duration of effect of the modified neurotoxin of the present invention relative to the identical unmodified neurotoxin. For example, a "decreased duration of effect" can be determined using the "Mouse Running Assay". The "Mouse Running Assay" is well-known to the person skilled in the art and measures the daily running distance of a mouse in a treadmill after a botulinum neurotoxin was injected into the M. gastrocnemius (see Keller JE. Recovery from botulinum neurotoxin poisoning in vivo. Neuroscience. 2006 May 12;139(2):629-37). The distance which a mouse is able to run in the treadmill the day before the botulinum neurotoxin is injected is used as comparison and is set as 100%. A daily running distance of no more than 80% of the initial running distance is regarded as paralysis of the muscle. If the treated mice show a paralysis of the muscle for less than 11 days, preferably less than 10 days, preferably less than 9 days, preferably less than 8 days, preferably less than 7 days post injection of a botulinum neurotoxin serotype A, the botulinum neurotoxin serotype A is considered to exhibit a "decreased duration of effects". In other words, if the treated mice need less than 80%, preferably less than 70%, preferably less than 60%, preferably less than 50%, preferably less than 40% of the time to achieve of 80% of the initial running distance in comparison to the mice treated with the unmodified botulinum neurotoxin serotype A than the botulinum neurotoxin serotype A is considered to exhibit a "decreased duration of effects".

In the context of the present invention, the term "faster onset of effect/action" is defined as a more than about 10%, particularly more than about 30%, more particularly more than about 50% faster onset of effect of the modified neurotoxin of the present invention relative to the identical neurotoxin without said modifications at the C-terminus of the light chain. For example, a "faster onset of effect" can be determined using the "Mouse Running Assay" if the maximum paralytic effect is attained earlier than three days post injection of a botulinum neurotoxin serotype A.

In the context of the present invention the term "denervation" refers to denervation resulting from administration of a chemodenervating agent, for example a neurotoxin.

In the context of the present invention, the term "localized denervation" or "localized paralysis" refers to denervation of a particular anatomical region, usually a muscle or a group of anatomically and/or physiologically related muscles, which results from administration of a chemodenervating agent, for example a neurotoxin, to the particular anatomical region.

Without wishing to be bound by theory, the recombinant botulinum neurotoxins of the present invention might show decreased biological half-life, increased degradation rates, accelerated uptake by neuronal cells, and/or modified intracellular translocation rates, in each case relative to an identical parental botulinum neurotoxin without said modifications at the C-terminus of the light chain..

In particular embodiments, the decreased duration of effect is due to a decreased biological half-life.

In the context of the present invention, the term "biological half-life" specifies the lifespan of a protein, for example of a botulinum neurotoxin serotype A, *in vivo.* In the context of the present invention, the term "biological half-life" refers to the period of time, by which half of a protein pool is degraded *in vivo.* For example it refers to the period of time, by which half of the amount of botulinum neurotoxin of one administered dosage is degraded.

In the context of the present invention, the term "decreased biological half-life" is defined as a more than about 20%, particularly more than about 50%, more particularly more than about 90% decreased biological half-life of the modified neurotoxin of the present invention relative to the identical neurotoxin without said modifications at the C-terminus of the light chain.

In the context of the present invention, the term "increased degradation rate" means that the modifications at the C- terminus of the light chain possibly enhances the degradation processes of the light chain in the cytosol of the neuron such as, for example, the attack of proteases or modifying enzymes like E3 ligases. Thus, the half-life of the light chain in the neuron is shortened resulting in a decreased duration of the therapeutic effect.

In particular embodiments, the recombinant botulinum neurotoxin is for the use in the treatment of a disease requiring improved chemodenervation, wherein the recombinant botulinum neurotoxin causes shorter lasting denervation relative to an identical botulinum neurotoxin without said modifications at the C-terminus of the light chain.

In another aspect, the present invention relates to a pharmaceutical or cosmetic composition comprising the recombinant botulinum neurotoxin of the present invention. For preparing a pharmaceutical preparation comprising a botulinum neurotoxin the toxin can be formulated by various techniques dependent on the desired application purposes which are known in the art. For example, the (biologically active) botulinum neurotoxin polypeptide can be used in combination with one or more pharmaceutically acceptable carriers as a pharmaceutical composition. The pharmaceutically acceptable carrier(s) must be acceptable in the sense of being compatible with the other ingredients of the formulation and being not deleterious to the recipient thereof. The pharmaceutical carrier employed may include a solid, a gel, or a liquid. Exemplary of solid carriers are lactose, terra alba, sucrose, talc, gelatine, agar, pectin, acacia, magnesium stearate, stearic acid and the like. Exemplary of liquid carriers are glycerol, phosphate buffered saline solution, water, emulsions, various types of wetting agents, and the like. Suitable carriers comprise those mentioned above and others well known in the art, see, e.g., Remington's Pharmaceutical Sciences, Mack Publishing Company, Easton, Pennsylvania. In an aspect, the pharmaceutical composition can be dissolved in a diluent, prior to administration. The diluent is also selected so as not to affect the biological activity of the Neurotoxin product. Examples of such diluents are distilled water or physiological saline. In addition, the pharmaceutical composition or formulation may also include other carriers or non-toxic, non-therapeutic, non-immunogenic stabilizers and the like. Thus, the formulated Neurotoxin product can be present, in an aspect, in liquid or lyophilized form. In an aspect, it can be present together with glycerol, protein stabilizers (HSA) or non-protein stabilizers such as polyvinyl pyrrolidone (PVP), hyaluronic acid or free amino acids. In an aspect, suitable non-proteinaceous stabilizers are disclosed in WO 2005/007185 or WO 2006/020208. The formulated Neurotoxin product may be used for human or animal therapy of various diseases or disorders in a therapeutically effective dose or for cosmetic purposes.

In particular embodiments, the recombinant botulinum neurotoxin of the present invention or the pharmaceutical composition of the present invention is for use in the treatment of a disease or condition taken from the list of:
wound healing, immobilisation for bone and tendon fracture treatment, post surgery immobilization, specifically in connection with haemorrhoidectomy, introduction of dental implants, or hip joint replacement (endoprothesis), epicondylitis, knee arthroplasty, ophthalmo logical surgery, acne, irritable bowel disease, vaginism, low back pain, or benign prostate dysplasia.

In yet another aspect, the present invention relates to the use of the composition of the present invention for cosmetic treatment.

In the context of the present invention, the term "cosmetic treatment" relates to uses in cosmetic or aesthetic applications, such as the treatment of wrinkles, crow's feet, glabella frown lines, reduction of the masseter muscle, reduction of the calves, removing of facial asymmetries etc..

In another aspect, the present invention relates to a pharmaceutical or cosmetic composition comprising the recombinant botulinum neurotoxin of the present invention together with wild type botulinum neurotoxin A. The composition shows a faster onset without decreased duration of effect relative to an identical botulinum neurotoxin without the modifications at the C-terminus of the light chain. In particular embodiments, the composition comprises the recombinant botulinum neurotoxin of the present invention together with wild type botulinum neurotoxin A in a dose mixing ratio of 3:1 to 1:3, preferably 3:2, 1:1 or 2:3.

In another aspect, the present invention relates to a method for the generation of the recombinant botulinum neurotoxin of the present invention, comprising the step of obtaining a recombinant nucleic acid sequence encoding a recombinant single-chain precursor botulinum neurotoxin by the modifying the nucleic acid sequence encoding a parental botulinum neurotoxin at the C-terminus of the light chain.

In the context of the present invention, the term "recombinant nucleic acid sequence" refers to a nucleic acid, which has been generated by joining genetic material from two different sources.

In the context of the present invention, the term "single-chain precursor botulinum neurotoxin" refers to a single-chain precursor for a disulfide-linked di-chain botulinum neurotoxin, comprising a functionally active botulinum neurotoxin light chain, a functionally active neurotoxin heavy chain, and a loop region linking the C-terminus of the light chain with the N-terminus of the heavy chain.
In the context of the present invention, the term "recombinant single-chain precursor botulinum neurotoxin" refers to a single-chain precursor botulinum neurotoxin comprising a modified C-terminus of the light chain of the neurotoxin.

In particular embodiments, the recombinant single-chain precursor botulinum neurotoxin comprises a protease cleavage site in said loop region.

Single-chain precursor botulinum neurotoxins have to be proteolytically cleaved to obtain the final biologically active botulinum neurotoxins. Proteolytic cleavage may either occur during heterologous expression by host cell enzymes, or by adding proteolytic enzymes to the raw protein material isolated after heterologous expression. Naturally occurring botulinum neurotoxins usually contain one or more cleavage signals for proteases which post-translationally cleave the single-chain precursor molecule, so that the final di- or multimeric complex can form. At present, botulinum neurotoxins are still predominantly produced by fermentation processes using appropriate Clostridium strains. During the fermentation process, the single-chain precursors are proteolytically cleaved by an unknown clostridial protease to obtain the biologically active di-chain clostridial neurotoxin. In cases, where the single-chain precursor molecule is the precursor of a protease, autocatalytic cleavage may occur. Alternatively, the protease can be a separate non-clostridial enzyme expressed in the same cell. WO 2006/076902 describes the proteolytic cleavage of a recombinant clostridial neurotoxin single-chain precursor at a heterologous recognition and cleavage site by incubation of the *E. coli* host cell lysate. The proteolytic cleavage is carried out by an unknown *E*. *coli* protease. In certain applications of recombinant expression, modified protease cleavage sites have been introduced recombinantly into the interchain region between the light and heavy chain of clostridial toxins, e.g. protease cleavage sites for human thrombin or non-human proteases (see WO 01/14570).

In particular embodiments, the protease cleavage site is a site that is cleaved by a protease selected from the list of: thrombin, trypsin, enterokinase, factor Xa, plant papain, insect papain, crustacean papain, enterokinase, human rhinovirus 3C protease, human enterovirus 3C protease, tobacco etch virus protease, Tobacco Vein Mottling Virus, subtilisin and caspase 3.

In a particular embodiment, the recombinant single-chain precursor botulinum neurotoxin further comprises a binding tag, particularly selected from the group comprising: glutathione-S-transferase (GST), maltose binding protein (MBP), a His-tag, a StrepTag, or a FLAG-tag.

In the context of the present invention, the term "parental botulinum neurotoxin" refers to an initial botulinum neurotoxin without modifications at the C-terminus of the light chain, selected from a natural botulinum neurotoxin, a functional variant of a natural botulinum neurotoxin or a chimeric botulinum neurotoxin.

In particular embodiments, the method for the generation of the recombinant botulinum neurotoxin of the present invention further comprises the step of heterologously expressing said recombinant nucleic acid sequence in a host cell, particularly in a bacterial host cell, more particularly in an *E*. *coli* host cell.

In certain embodiments, the *E. coli* cells are selected from *E. coli* XL1-Blue, Nova Blue, TOP10, XL10-Gold, BL21, and K12.

In particular embodiments, the method for the generation of the recombinant botulinum neurotoxin of the present invention additionally comprises at least one of the steps of (i) generating a disulfide-linked di-chain recombinant botulinum neurotoxin comprising a modified C- terminus of the light chain by causing or allowing contacting of said recombinant single-chain precursor botulinum neurotoxin with an endoprotease and (ii) purification of said recombinant single-chain precursor botulinum neurotoxin or said disulfide-linked di-chain recombinant botulinum neurotoxin by chromatography.

In particular embodiments, the recombinant single-chain precursor botulinum neurotoxin, or the recombinant disulfide-linked di-chain botulinum neurotoxin, is purified after expression, or in the case of the recombinant disulfide-linked di-chain botulinum neurotoxin, after the cleavage reaction. In particular such embodiments, the protein is purified by chromatography, particularly by immunoaffinity chromatography, or by chromatography on an ion exchange matrix, a hydrophobic interaction matrix, or a multimodal chromatography matrix, particularly a strong ion exchange matrix, more particularly a strong cation exchange matrix.

In the context of the present invention, the term "causing ... contacting of said recombinant single-chain precursor botulinum neurotoxin ...with an endoprotease" refers to an active and/or direct step of bringing said neurotoxin and said endoprotease in contact, whereas the term "allowing contacting of a recombinant single-chain precursor botulinum neurotoxin ...with an endoprotease" refers to an indirect step of establishing conditions in such a way that said neurotoxin and said endoprotease are getting in contact to each other.

In the context of the present invention, the term "endoprotease" refers to a protease that breaks peptide bonds of non-terminal amino acids (i.e. within the polypeptide chain). As they do not attack terminal amino acids, endoproteases cannot break down peptides into monomers.

In particular embodiments, cleavage of the recombinant single-chain precursor botulinum neurotoxin is near-complete.

In the context of the present invention, the term "near-complete" is defined as more than about 95% cleavage, particularly more than about 97.5%, more particularly more than about 99% as determined by SDS-PAGE and subsequent Western Blot or reversed phase chromatography.

In particular embodiments, cleavage of the recombinant single-chain precursor botulinum neurotoxin occurs at a heterologous cleavage signal located in the loop region of the recombinant precursor botulinum neurotoxin.

In particular embodiments, the cleavage reaction is performed with crude host cell lysates containing said single-chain precursor protein.

In other particular embodiments, the single-chain precursor protein is purified or partially purified, particularly by a first chromatographic enrichment step, prior to the cleavage reaction.

In the context of the present invention, the term "purified" relates to more than about 90% purity. In the context of the present invention, the term "partially purified" relates to purity of less than about 90% and an enrichment of more than about two fold.

In another aspect, the present invention relates to a recombinant single-chain botulinum neurotoxin, which is a precursor for the recombinant botulinum neurotoxin of the present invention Thus, in such aspect, the present invention relates to a recombinant single-chain precursor botulinum neurotoxin comprising a modified C-terminus of the light chain at position 419 to 438.

In another aspect, the present invention relates to a method for obtaining the nucleic acid sequence of the present invention, comprising the step of modifying a nucleic acid sequence encoding a parental botulinum neurotoxin.

In another aspect, the present invention relates to a vector comprising the nucleic acid sequence of the present invention, or the nucleic acid sequence obtainable by the method of the present invention.

In another aspect, the present invention relates to a recombinant host cell comprising the nucleic acid sequence of the present invention, the nucleic acid sequence obtainable by the method of the present invention, or the vector of the present invention.

In certain embodiments, the recombinant host cells are selected from *E. coli* XL1 -Blue, Nova Blue, TOP10, XL10-Gold, BL21, and K12.

In another aspect, the present invention relates to a method for producing the recombinant single-chain precursor botulinum neurotoxin of the present invention, comprising the step of expressing the nucleic acid sequence of the present invention, or the nucleic acid sequence obtainable by the method of the present invention, or the vector of the present invention in a recombinant host cell, or cultivating the recombinant host cell of the present invention under conditions that result in the expression of said nucleic acid sequence.

### EXAMPLES

### Example 1: Generation and Purification of a BoNT/A with modifications T420R, F423M and Y426F (rBoNT/A-AL5) Molecular biology.

### Molecular biology

For expression of BoNT/A in *E.coli* codon optimized gene constructs were cloned into pET29c (dna and protein sequence table TS1). These variants contain C-terminal fused His6- and Strep-affinity tags which can be cleaved after protein purification *via* thrombin. In order to construct a plasmid for the expression of BonT/A-AL5 a synthetic gene (Thermo Scientific) with the corresponding mutations of (T420R F423M Y426F) in the BoNT/A1 wild type sequence (SEQ ID NO 1) and restriction sites Sbfl and ScaI was used.

### Protein expression and purification.

Expression of rBoNT/A variants was performed in Riesenberg media with 50 µg/mL Kanamycin{Riesenberg, 1991 #1}. Cells were grown in shake flasks (37°C, 175 r.p.m) until an OD600 of 1.5-2 was reached. For induction of protein expression 1 mM IPTG (Fermentas) was added to the E.coli culture. Protein synthesis was performed for 24 h (15 °C, 175 r.p.m.). Cells were collected by centrifugation (5,000 r.p.m., 20 min, 4 °C) and resuspended in His binding buffer pH 8.0 (50 mM Tris, 150 mM NaCl, 5 mM Imidazol) containing EDTA-free protease inhibitor complete (Roche Diagnostics). For the determination of endopeptidase activity and *in vivo* characterization, the different toxin variants were extracted and purified. Resuspended pellets were disrupted in 2-3 cycles by a French Press Cell Disrupter (Thermo Electron Corporation) at 4 °C. The resulting crude extracts were centrifuged (20,000 r.p.m., 30 min, 4 °C), and the supernatants with the soluble proteins were recovered. Protein purification was carried out by fast protein liquid chromatography (GE Healthcare) using a three step purification protocols. The first capture step was performed by IMAC using a HisTrap HP 1 mL column (GE Healthcare). The column was washed (1 ml min-1 working flow) using a two-step protocol with His elution buffer (50 mM Tris, 150 mM NaCl, 400 mM Imidazol pH 8,0). The elution of the toxin proteins occurred at 400 mM Imidazol. In a further step a Strep-Tactin affinity chromatography was performed as previously described (IBA GmbH). As an alternative instead of a second affinity chromatography a cation exchange chromatography with a HiTrap SP HP 1 mL column (GE Healthcare, Freiburg, Germany) was used. The corresponding samples were diluted with SP binding buffer (50 mM Tris, pH 8) and eluted with SP elution buffer (50 mM Tris, 1 M NaCl, pH 8). This procedure was followed by a SEC using a Superdex 200 10/300 column (GE Healthcare). The SEC running buffer (20 mM Tris, 150 mM NaCl, 2,5 mM CaCl2 pH 7,7) was also used to store the purified protein solutions in aliquots at -20 °C. Each protein was analyzed by applying 0,5-1 µg on 4-12% gradient SDS-PAGE (Novex Life Technologies) and stained with Coomassie G-250 based SimplyBlue safe stain (Pierce). Each protein was judged >98% pure before applying *in vitro* or *in vivo* experiments.

### Thrombin cleavage

BoNT/A preparations were activated with Thrombin (Merck Millipore; 8 U/1 mg BoNT) for 24 h at 20 °C yielding > 99% of di-chain toxin. Afterwards the cleavage protease was eliminated with the previously described Strep-Tactin Kit (IBA GmbH).

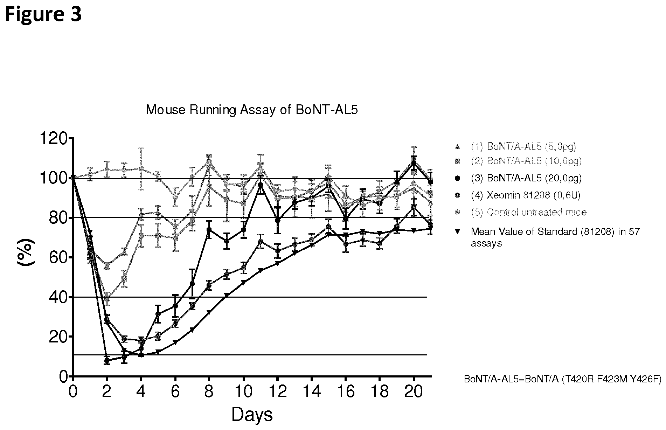

was performed in expression strain *E. coli* BI21. Purification was done using a combination of his affinity, ion exchange and size exclusion chromatography, followed by activation using thrombin. Figure 3 summarizes the results of purification and activation.

### Example 2: Duration of Effect of BoNT/A-A5 in the "Mouse Running Assay"

Three different dosages of BoNT/A-AL5, i.e. 5,0 pg, 10,0 pg and 20,0 pg were injected into the M. gastrocnemius of each mice in comparison to standard Xeomin® (0.6U). The mice had been trained in a treadmill. The daily running distance in the treadmill was measured over 21 days. The paralysis caused by the toxins was plotted as percentage of the running distance on the day before the injection, which was set as 100%, against the time (see Figure 3).

The maximum paralytic effect was attained after 2 days post injection of a dose (20pg/mouse) equipotent to Xeomin® whereas Xeomin achieved maximum paralysis at day 4. The Duration of paralytic effect of BoNT/A-A5 was markedly shorter when compared with the Xeomin®.

### Example 3: Generation and Purification of a BoNT/A with mutations T420E, F423M and Y426F (BoNT/A-AL6)

### Molecular Biology

For expression of BoNT/A1 in E.coli codon optimized gene constructs were cloned into pET29c (dna and protein sequence table TS1). These variants contain C-terminal fused His6- and Strep-affinity tags which can be cleaved after protein purification via thrombin. In order to construct a plasmid for the expression of BonT/A-AL5 a synthetic gene (Thermo Scientific) with the corresponding mutations of (T420R F423M Y426F) in the BoNT/A1 wild type sequence (SEQ ID NO 1) and restriction sites Sbfl and ScaI was used. For site directed mutagenesis to prepare BoNT/A-AL6 (T420E F423M Y426F) standard QuickChange II XL (Agilent Technologies) protocol was applied. The corresponding primers and templates are listed in table TS2.

### Protein expression and purification.

Expression of rBoNT/A variants was performed in Riesenberg media with 50 µg/mL Kanamycin{Riesenberg, 1991 #1}. Cells were grown in shake flasks (37°C, 175 r.p.m) until an OD600 of 1.5-2 was reached. For induction of protein expression 1 mM IPTG (Fermentas) was added to the E.coli culture. Protein synthesis was performed for 24 h (15 °C, 175 r.p.m.). Cells were collected by centrifugation (5,000 r.p.m., 20 min, 4 °C) and resuspended in His binding buffer pH 8.0 (50 mM Tris, 150 mM NaCl, 5 mM Imidazol) containing EDTA-free protease inhibitor complete (Roche Diagnostics). For the determination of endopeptidase activity and *in vivo* characterization, the different toxin variants were extracted and purified. Resuspended pellets were disrupted in 2-3 cycles by a French Press Cell Disrupter (Thermo Electron Corporation) at 4 °C. The resulting crude extracts were centrifuged (20,000 r.p.m., 30 min, 4 °C), and the supernatants with the soluble proteins were recovered. Protein purification was carried out by fast protein liquid chromatography (GE Healthcare) using a three step purification protocols. The first capture step was performed by IMAC using a HisTrap HP 1 mL column (GE Healthcare). The column was washed (1 ml min-1 working flow) using a two-step protocol with His elution buffer (50 mM Tris, 150 mM NaCl, 400 mM Imidazol pH 8,0). The elution of the toxin proteins occurred at 400 mM Imidazol. In a further step a Strep-Tactin affinity chromatography was performed as previously described (IBA GmbH). As an alternative instead of a second affinity chromatography a cation exchange chromatography with a HiTrap SP HP 1 mL column (GE Healthcare, Freiburg, Germany) was used. The corresponding samples were diluted with SP binding buffer (50 mM Tris, pH 8) and eluted with SP elution buffer (50 mM Tris, 1 M NaCl, pH 8). This procedure was followed by a SEC using a Superdex 200 10/300 column (GE Healthcare). The SEC running buffer (20 mM Tris, 150 mM NaCl, 2,5 mM CaCl2 pH 7,7) was also used to store the purified protein solutions in aliquots at -20 °C. Each protein was analyzed by applying 0,5-1 µg on 4-12% gradient SDS-PAGE (Novex Life Technologies) and stained with Coomassie G-250 based SimplyBlue safe stain (Pierce). Each protein was judged >98% pure before applying *in vitro* or *in vivo* experiments.

### Thrombin cleavage

BoNT/A-A6 preparations was activated with Thrombin (Merck Millipore; 8 U/1 mg BoNT) for 24 h at 20 °C yielding > 99% of di-chain toxin. Afterwards the cleavage protease was eliminated with the previously described Strep-Tactin Kit (IBA GmbH).

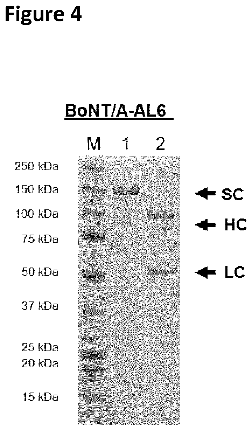

was performed in expression strain *E. coli* BI21. Purification was done using a combination of his affinity, ion exchange and size exclusion chromatography, followed by activation using thrombin. Figure 4 summarizes the results of purification and activation.

### Example 4: Duration of Effect of BoNT/A-A6 "Mouse Running Assay"

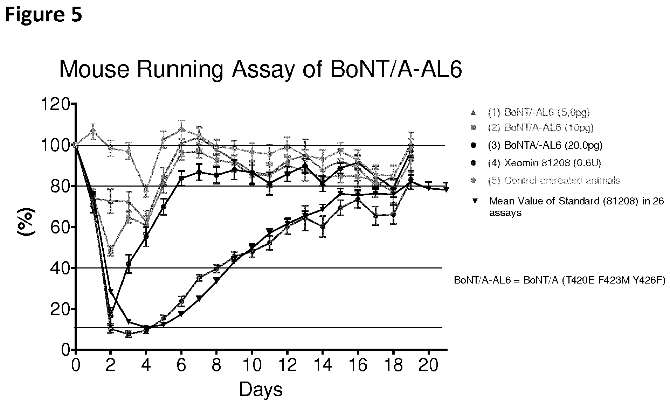

Three different dosages of B0NT/A-AL6, i.e. 5,0 pg, 10,0 pg and 20,0 pg were injected into the M. gastrocnemius of each mice in comparison to standard Xeomin® (0.6 U). The mice had been trained in a treadmill. The daily running distance in the treadmill was measured over 21 days. The paralysis caused by the toxins was plotted as percentage of the running distance on the day before the injection, which was set as 100%, against the time (see Figure 5).

The maximum paralytic effect was attained after 2 days post injection of a dose (20pg/mouse) equipotent to Xeomin® whereas Xeomin achieved maximum paralysis at day 3-4. The duration of paralytic effect of BoNT/A-A6 was markedly shorter when compared with the Xeomin®: after 6 days mice treated with BoNT/A-AL6 were already running a distance of 80% of the initial running distance whereas mice treated with Xeomin® needed about 14 days to achieve this distance.

### Table 1: Sequences

SEQ ID NO 1: (recombinant BoNT A including His.tag
SEQ ID NO 2: (recombinant BoNT A with T420R, F423M, Y426F including His.tag)
SEQ ID NO 3: (recombinant BoNT A with T420E, F423M, Y426F including His.tag)
SEQ ID NO 4: (recombinant BoNT A with T420E, Y426F, R432G, K438R including His.tag)
SEQ ID NO 1: 
MPFVNKQFNYKDPVNGVDIAYIKIPNAGQMQPVKAFKIHNKIWVIPERDTFTNPEEGDLN
PPPEAKQVPVSYYDSTYLSTDNEKDNYLKGVTKLFERIYSTDLGRMLLTSIVRGIPFWGG
STIDTELKVIDTNCINVIQPDGSYRSEELNLVIIGPSADIIQFECKSFGHEVLNLTRNGY
GSTQYIRFSPDFTFGFEESLEVDTNPLLGAGKFATDPAVTLAHELIHAGHRLYGIAINPN
RVFKVNTNAYYEMSGLEVSFEELRTFGGHDAKFIDSLQENEFRLYYYNKFKDIASTLNKA
KSIVGTTASLQYMKNVFKEKYLLSEDTSGKFSVDKLKFDKLYKMLTEIYTEDNFVKFFKV
LNRKTYLNFDKAVFKINIVPKVNYTIYDGFNLRNTNLAANFNGQNTEINNMNFTKLKNFT
GLFEFYKLLCVRGIITSKAGAGKSLVPRGSAGAGALNDLCIKVNNWDLFFSPSEDNFTND
LNKGEEITSDTNIEAAEENISLDLIQQYYLTFNFDNEPENISIENLSSDIIGQLELMPNI
ERFPNGKKYELDKYTMFHYLRAQEFEHGKSRIALTNSVNEALLNPSRVYTFFSSDYVKKV
NKATEAAMFLGWVEQLVYDFTDETSEVSTTDKIADITIIIPYIGPALNIGNMLYKDDFVG
ALIFSGAVILLEFIPEIAIPVLGTFALVSYIANKVLTVQTIDNALSKRNEKWDEVYKYIV
TNWLAKVNTQIDLIRKKMKEALENQAEATKAIINYQYNQYTEEEKNNINFNIDDLSSKLN
ESINKAMININKFLNQCSVSYLMNSMIPYGVKRLEDFDASLKDALLKYIYDNRGTLIGQV
DRLKDKVNNTLSTDIPFQLSKYVDNQRLLSTFTEYIKNIINTSILNLRYESNHLIDLSRY
ASKINIGSKVNFDPIDKNQIQLFNLESSKIEVILKNAIVYNSMYENFSTSFWIRIPKYFN
SISLNNEYTIINCMENNSGWKVSLNYGEIIWTLQDTQEIKQRVVFKYSQMINISDYINRW
IFVTITNNRLNNSKIYINGRLIDQKPISNLGNIHASNNIMFKLDGCRDTHRYIWIKYFNL
FDKELNEKEIKDLYDNQSNSGILKDFWGDYLQYDKPYYMLNLYDPNKYVDVNNVGIRGYM
YLKGPRGSVMTTNIYLNSSLYRGTKFIIKKYASGNKDNIVRNNDRVYINVVVKNKEYRLA
TNASQAGVEKILSALEIPDVGNLSQVVVMKSKNDQGITNKCKMNLQDNNGNDIGFIGFHQ
 
FNNIAKLVASNWYNRQIERSSRTLGCSWEFIPVDDGWGERPLGDLVPRGSANSSSVDKLW

SHPQFEKLEHHHHHH
SEQ ID NO 2: 
MPFVNKQFNYKDPVNGVDIAYIKIPNAGQMQPVKAFKIHNKIWVIPERDTFTNPEEGDLN
PPPEAKQVPVSYYDSTYLSTDNEKDNYLKGVTKLFERIYSTDLGRMLLTSIVRGIPFWGG
STIDTELKVIDTNCINVIQPDGSYRSEELNLVIIGPSADIIQFECKSFGHEVLNLTRNGY
GSTQYIRFSPDFTFGFEESLEVDTNPLLGAGKFATDPAVTLAHELIHAGHRLYGIAINPN
RVFKVNTNAYYEMSGLEVSFEELRTFGGHDAKFIDSLQENEFRLYYYNKFKDIASTLNKA
KSIVGTTASLQYMKNVFKEKYLLSEDTSGKFSVDKLKFDKLYKMLTEIYTEDNFVKFFKV
LNRKTYLNFDKAVFKINIVPKVNYTIYDGFNLRNTNLAANFNGQNTEINNMNFTKLKNFR
GLMEFFKLLCVRGIITSKAGAGKSLVPRGSAGAGALNDLCIKVNNWDLFFSPSEDNFTND
LNKGEEITSDTNIEAAEENISLDLIQQYYLTFNFDNEPENISIENLSSDIIGQLELMPNI
ERFPNGKKYELDKYTMFHYLRAQEFEHGKSRIALTNSVNEALLNPSRVYTFFSSDYVKKV
NKATEAAMFLGWVEQLVYDFTDETSEVSTTDKIADITIIIPYIGPALNIGNMLYKDDFVG
ALIFSGAVILLEFIPEIAIPVLGTFALVSYIANKVLTVQTIDNALSKRNEKWDEVYKYIV
TNWLAKVNTQIDLIRKKMKEALENQAEATKAIINYQYNQYTEEEKNNINFNIDDLSSKLN
ESINKAMININKFLNQCSVSYLMNSMIPYGVKRLEDFDASLKDALLKYIYDNRGTLIGQV
DRLKDKVNNTLSTDIPFQLSKYVDNQRLLSTFTEYIKNIINTSILNLRYESNHLIDLSRY
ASKINIGSKVNFDPIDKNQIQLFNLESSKIEVILKNAIVYNSMYENFSTSFWIRIPKYFN
SISLNNEYTIINCMENNSGWKVSLNYGEIIWTLQDTQEIKQRVVFKYSQMINISDYINRW
IFVTITNNRLNNSKIYINGRLIDQKPISNLGNIHASNNIMFKLDGCRDTHRYIWIKYFNL
FDKELNEKEIKDLYDNQSNSGILKDFWGDYLQYDKPYYMLNLYDPNKYVDVNNVGIRGYM
 
YLKGPRGSVMTTNIYLNSSLYRGTKFIIKKYASGNKDNIVRNNDRVYINVVVKNKEYRLA

TNASQAGVEKILSALEIPDVGNLSQVVVMKSKNDQGITNKCKMNLQDNNGNDIGFIGFHQ

FNNIAKLVASNWYNRQIERSSRTLGCSWEFIPVDDGWGERPLGDLVPRGSANSSSVDKLW

SHPQFEKLEHHHHHH
SEQ ID NO 3: 
MPFVNKQFNYKDPVNGVDIAYIKIPNAGQMQPVKAFKIHNKIWVIPERDTFTNPEEGDLN
PPPEAKQVPVSYYDSTYLSTDNEKDNYLKGVTKLFERIYSTDLGRMLLTSIVRGIPFWGG
STIDTELKVIDTNCINVIQPDGSYRSEELNLVIIGPSADIIQFECKSFGHEVLNLTRNGY
GSTQYIRFSPDFTFGFEESLEVDTNPLLGAGKFATDPAVTLAHELIHAGHRLYGIAINPN
RVFKVNTNAYYEMSGLEVSFEELRTFGGHDAKFIDSLQENEFRLYYYNKFKDIASTLNKA
KSIVGTTASLQYMKNVFKEKYLLSEDTSGKFSVDKLKFDKLYKMLTEIYTEDNFVKFFKV
LNRKTYLNFDKAVFKINIVPKVNYTIYDGFNLRNTNLAANFNGQNTEINNMNFTKLKNFE
GLMEFFKLLCVRGIITSKAGAGKSLVPRGSAGAGALNDLCIKVNNWDLFFSPSEDNFTND
LNKGEEITSDTNIEAAEENISLDLIQQYYLTFNFDNEPENISIENLSSDIIGQLELMPNI
ERFPNGKKYELDKYTMFHYLRAQEFEHGKSRIALTNSVNEALLNPSRVYTFFSSDYVKKV
NKATEAAMFLGWVEQLVYDFTDETSEVSTTDKIADITIIIPYIGPALNIGNMLYKDDFVG
ALIFSGAVILLEFIPEIAIPVLGTFALVSYIANKVLTVQTIDNALSKRNEKWDEVYKYIV
TNWLAKVNTQIDLIRKKMKEALENQAEATKAIINYQYNQYTEEEKNNINFNIDDLSSKLN
ESINKAMININKFLNQCSVSYLMNSMIPYGVKRLEDFDASLKDALLKYIYDNRGTLIGQV
DRLKDKVNNTLSTDIPFQLSKYVDNQRLLSTFTEYIKNIINTSILNLRYESNHLIDLSRY
ASKINIGSKVNFDPIDKNQIQLFNLESSKIEVILKNAIVYNSMYENFSTSFWIRIPKYFN
 
SISLNNEYTIINCMENNSGWKVSLNYGEIIWTLQDTQEIKQRVVFKYSQMINISDYINRW
IFVTITNNRLNNSKIYINGRLIDQKPISNLGNIHASNNIMFKLDGCRDTHRYIWIKYFNL
FDKELNEKEIKDLYDNQSNSGILKDFWGDYLQYDKPYYMLNLYDPNKYVDVNNVGIRGYM
YLKGPRGSVMTTNIYLNSSLYRGTKFIIKKYASGNKDNIVRNNDRVYINVVVKNKEYRLA
TNASQAGVEKILSALEIPDVGNLSQVVVMKSKNDQGITNKCKMNLQDNNGNDIGFIGFHQ
FNNIAKLVASNWYNRQIERSSRTLGCSWEFIPVDDGWGERPLGDLVPRGSANSSSVDKLW
SHPQFEKLEHHHHHH
SEQ ID NO4:

MPFVNKQFNYKDPVNGVDIAYIKIPNAGQMQPVKAFKIHNKIWVIPERDTFTNPEEGDLN
PPPEAKQVPVSYYDSTYLSTDNEKDNYLKGVTKLFERIYSTDLGRMLLTSIVRGIPFWGG
STIDTELKVIDTNCINVIQPDGSYRSEELNLVIIGPSADIIQFECKSFGHEVLNLTRNGY
GSTQYIRFSPDFTFGFEESLEVDTNPLLGAGKFATDPAVTLAHELIHAGHRLYGIAINPN
RVFKVNTNAYYEMSGLEVSFEELRTFGGHDAKFIDSLQENEFRLYYYNKFKDIASTLNKA
KSIVGTTASLQYMKNVFKEKYLLSEDTSGKFSVDKLKFDKLYKMLTEIYTEDNFVKFFKV
LNRKTYLNFDKAVFKINIVPKVNYTIYDGFNLRNTNLAANFNGQNTEINNMNFTKLKNFS
GLFEFFKLLCVGGIITSRAGAGKSLVPRGSAGAGALNDLCIKVNNWDLFFSPSEDNFTND
LNKGEEITSDTNIEAAEENISLDLIQQYYLTFNFDNEPENISIENLSSDIIGQLELMPNI
ERFPNGKKYELDKYTMFHYLRAQEFEHGKSRIALTNSVNEALLNPSRVYTFFSSDYVKKV
NKATEAAMFLGWVEQLVYDFTDETSEVSTTDKIADITIIIPYIGPALNIGNMLYKDDFVG
ALIFSGAVILLEFIPEIAIPVLGTFALVSYIANKVLTVQTIDNALSKRNEKWDEVYKYIV
TNWLAKVNTQIDLIRKKMKEALENQAEATKAIINYQYNQYTEEEKNNINFNIDDLSSKLN

ESINKAMININKFLNQCSVSYLMNSMIPYGVKRLEDFDASLKDALLKYIYDNRGTLIGQV

DRLKDKVNNTLSTDIPFQLSKYVDNQRLLSTFTEYIKNIINTSILNLRYESNHLIDLSRY

ASKINIGSKVNFDPIDKNQIQLFNLESSKIEVILKNAIVYNSMYENFSTSFWIRIPKYFN

SISLNNEYTIINCMENNSGWKVSLNYGEIIWTLQDTQEIKQRVVFKYSQMINISDYINRW

IFVTITNNRLNNSKIYINGRLIDQKPISNLGNIHASNNIMFKLDGCRDTHRYIWIKYFNL

FDKELNEKEIKDLYDNQSNSGILKDFWGDYLQYDKPYYMLNLYDPNKYVDVNNVGIRGYM

YLKGPRGSVMTTNIYLNSSLYRGTKFIIKKYASGNKDNIVRNNDRVYINVVVKNKEYRLA

TNASQAGVEKILSALEIPDVGNLSQVVVMKSKNDQGITNKCKMNLQDNNGNDIGFIGFHQ

FNNIAKLVASNWYNRQIERSSRTLGCSWEFIPVDDGWGERPLGDLVPRGSANSSSVDKLW

SHPQFEKLEHHHHHH

## Claims

1. A recombinant botulinum neurotoxin serotype A or a functional variant thereof comprising at least one amino acid modification, wherein said at least one amino acid modification is located at the C-terminus of the light chain at position 419 to 438 (SEQ ID NO 1), wherein said at least one amino acid modification confers a faster onset and/or a shorter duration of effect than an otherwise identical botulinum neurotoxin lacking said at least one amino acid modification.

2. The recombinant neurotoxin of claim 1, wherein said at least one amino acid modification is located at the C-terminus of the light chain at at least one position selected from F419, T420, F423, Y426, R432 and K438.

3. The recombinant neurotoxin of claim 1 or 2, wherein the recombinant neurotoxin comprises at least two or three or four or five amino acid modifications.

4. The recombinant neurotoxin of any one of claim 1 to 3, wherein three amino acid modifications are located at the C-terminus of the light chain at position T420, F423 and Y426.

5. The recombinant neurotoxin of any one of claim 1 to 4, wherein three amino acid modifications are located at the C-terminus of the light chain at position T420, F423 and Y426, wherein these amino acids are substituted as follows T420R, F423M and Y426F.

6. The recombinant neurotoxin of claim 1 to 4, wherein three amino acid modifications are located at the C-terminus of the light chain at position T420, F423 and Y426, wherein these amino acids are substituted as follows T420E, F423M and Y426F.

7. The recombinant neurotoxin of claim 1 to 3, wherein four amino acid modifications are located at the C-terminus of the light chain at position T420, Y426, R432, and K438.

8. The recombinant neurotoxin of claim 1 to 3, wherein four amino acid modifications are located at the C-terminus of the light chain at position T420, Y426, R432, and K438, wherein these amino acids are substituted as follows T420S, Y426F, R432G and K438R.

9. The recombinant neurotoxin of any one of claims 1 to 8 for the use in the treatment of a disease requiring improved chemodenervation, wherein the recombinant neurotoxin causes faster onset and/or shorter lasting denervation relative to an identical neurotoxin without said modification of the light chain.

10. A composition comprising the recombinant neurotoxin of any one of claims 1 to 8.

11. A pharmaceutical composition comprising the recombinant neurotoxin of any one of claims 1 to 8.

12. A pharmaceutical composition comprising the recombinant neurotoxin of any one of claims 1 to 8 together with the wild type botulinum neurotoxin A.

13. Use of the recombinant neurotoxin of any one of claims 1 to 8 for cosmetic treatment.

14. A method for the generation of a recombinant neurotoxin according to any one of claims 1 to 8, comprising the step of obtaining a recombinant nucleic acid sequence encoding a recombinant single-chain precursor neurotoxin by modification of the nucleic acid sequence encoding a parental neurotoxin, or wherein said method further comprises the step of heterologously expressing said recombinant nucleic acid sequence in a host cell, particularly in a bacterial host cell, more particularly in an *E*. *coli* host cell.

15. A recombinant single-chain neurotoxin, which is a precursor for the recombinant neurotoxin of any one of claims 1 to 8.
